# EUROPÄISCHE PATENTSCHRIFT

(11) **EP 0 555 242 B1**
(45) Veröffentlichungstag und Bekanntmachung des Hinweises auf die Patenterteilung: **04.01.1995**
(21) Anmeldenummer: 91918007.5
(22) Anmeldetag: 31.10.1991
(51) Int. Cl.: A61M 16/00

(54) **VORRICHTUNG ZUM ANWÄRMEN UND ANFEUCHTEN VON GASEN, INSBESONDERE VON ATEMGASEN BEI KÜNSTLICHER BEATMUNG**
DEVICE FOR HEATING AND MOISTENING GASES, ESPECIALLY RESPIRATORY GASES IN ARTIFICIAL RESPIRATION
DISPOSITIF DE CHAUFFAGE ET D'HUMIDIFICATION DE GAZ, NOTAMMENT DE GAZ RESPIRABLES LORS DE LA RESPIRATION ARTIFICIELLE

(30) Priorität: 02.11.1990 DE 4034969; 06.08.1991 DE 4126028
(43) Veröffentlichungstag der Anmeldung: 18.08.1993
(73) Patentinhaber: Lang, Volker, Prof. Dr., D-82131 Gauting (DE)
(72) Erfinder: Lang, Volker, Prof. Dr., D-82131 Gauting (DE)
(74) Vertreter: Laufhütte, Dieter, Dr.-Ing.
(86) Internationale Anmeldenummer: EP9102060
(87) Internationale Veröffentlichungsnummer: WO9207601

(56) Entgegenhaltungen:
- EP-A- 0 258 928
- EP-A- 0 413 127
- FR-A- 2 250 542
- FR-A- 2 636 845
- US-A- 3 912 795

## Beschreibung

Die Erfindung betrifft eine Vorrichtung zum Anwärmen und Anfeuchten von Gasen, insbesondere von Atemgasen bei der künstlichen Beatmung bestehend aus einer Kombination von mindestens einem passiven Wärme-Feuchte-Austauscher und mindestens einer aktiven Anwärm- und Anfeuchtvorrichtung.

Unter physiologischen Bedinungen dient die Nase der aktiven Anwärmung und Befeuchtung der Atemluft. Beim künstlich beatmeten Patienten wird diese jedoch durch einen Tubus (Schlauch) überbrückt, der mit seinem Ende in der Luftröhre liegt. Die für eine ungestörte Lungenfunktion unbedingt notwendige Befeuchtung und Erwärmung der Atemgase wird heute durch Vorrichtungen bewerkstelligt, die vor allem nach zwei unterschiedlichen Prinzipien arbeiten. Die Vorrichtung einer ersten Gattung führen der Atemluft aktiv Wärme und Feuchte zu. Dabei wird beispielsweise die trockene und kalte Atemluft durch einen elektrisch beheizten Wasserbad-Anfeuchter geführt und dabei konditioniert, bevor sie dem Patienten zugeführt wird. Die Vorrichtungen der zweiten Gattung arbeiten passiv als Warme-Feuchte-Austauscher (heat and moisture exchanger, H.M.E.). Hier wird Wärme und Feuchte der körperwarmen, feuchten Ausatemluft entzogen, die dann an die kalte, trockene Einatemluft abgegeben wird, ohne daß eine zusätzliche aktive Wärme- und Feuchtezufuhr von außen erfolgt.

Nach diesem Stand der Technik können zwar die mit einer aktiven Zufuhr von Wärme und Feuchte arbeitenden Vorrichtung hinreichend temperierte und befeuchtete Atemgase liefern, jedoch ist hierzu ein sehr hoher technischer und pflegerischer Aufwand notwendig, so daß die Entstehungs- und Betriebskosten derartiger Geräte sehr hoch sind. Im Gegensatz hierzu ist dieser Aufwand bei den einfach konstruierten und einfach anwendbaren passiven Wärme-Feuchte-Austauschern gering. Dafür erbringen aber diese bislang noch keine befriedigende Befeuchtungs- und Anwärmleistung für die künstliche Beatmung.

Die nicht vorveröffentlichte europäischen Patentanmeldung EP-A-0 413 127 A2 des gleichen Anmelders beschreibt eine gattungsgemäße Vorrichtung zum Anwärmen und Anfeuchten von Atemgasen bei künstlicher Beatmung bekannt, bei der die vorgenannten Vorteile durch die Kombination unabhängiger passiver Wärme-Feuchte-Austauscher mit einem aktiven Wärme-Feuchte-Austauscher kombiniert wurden. Jedoch ist diese Vorrichtung verhältnismäßig komplex aufgebaut. Darüber hinaus zeigt diese Vorrichtung teilweise Probleme mit einem zu großen Totraumvolumen, das sich durch Rückatmung von verbrauchter Atemluft besonders stark bei der Neugeborenenbeatmung wegen der hier auftretenden kleinen Atemzugvolumina auswirkt.

Aufgabe der vorliegenden Erfindung ist es demnach, eine gattungsgemäße Vorrichtung derart weiterzubilden, daß sie optimal befeuchtete Atemgase bei kleineren Totraumvolumina liefert und dabei noch möglichst einfach baut und eine einfache Bedienung ermöglicht.

Erfindungsgemäß wird diese Aufgabe ausgehend von einer gattungsgemäßen Vorrichtung durch die Merkmale des Kennzeichens des Anspruchs 1 gelöst. Erfindungsgemäß wird demnach ein in ein Gehäuse eingesetzter Wärme-Feuchte-Austauscher-Einsatz vorgesehen, der zu einem Teil als passiver Wärme-Feuchte-Austauscher arbeitet und zum anderen Teil zusammen mit einer ihn umgebenden Anwärm- und Anfeuchtvorrichtung als aktiver Wärme-Feuchte-Austauscher arbeitet. Hierdurch wird eine räumliche Einheit eines passiven Feuchte-Wärme-Austauschers und einer aktiven Anwärm- und Anfeuchtvorrichtung erzielt. Die Vorteile beider Prinzipien werden also in baulich kompakter Form in einer Vorrichtung vereinigt, so daß einerseits eine optimale Befeuchtung und Anwärmung der Atemgase bei gleichzeitig geringem technischem, pflegerischem und finanziellem Aufwand erzielt wird.

In vorteilhafter Weise ist die Vorrichtung derart ausgestaltet, daß ihr Gehäuse zweigeteilt ist, wobei der obere Gehäuseteil als konisches Verbindungsstück mit einem zylindrischen Hohlraum zur Aufnahme des passiven Wärme-Feuchte-Austauscher-Einsatzes gebildet ist und wobei das untere Gehäuseteil als aktive Befeuchtungs-Anwärm-Vorrichtung ausgebildet ist, welches einen Tubusadapter einsetzbar aufnehmen kann.

Weitere bevorzugte Ausführungsformen der Erfindung werden in den sich anschließenden Unteransprüchen widergegeben.

In Folge wird die Erfindung anhand eines in der Zeichnung dargestellten Ausführungsbeispieles näher erläutert. Es zeigen:

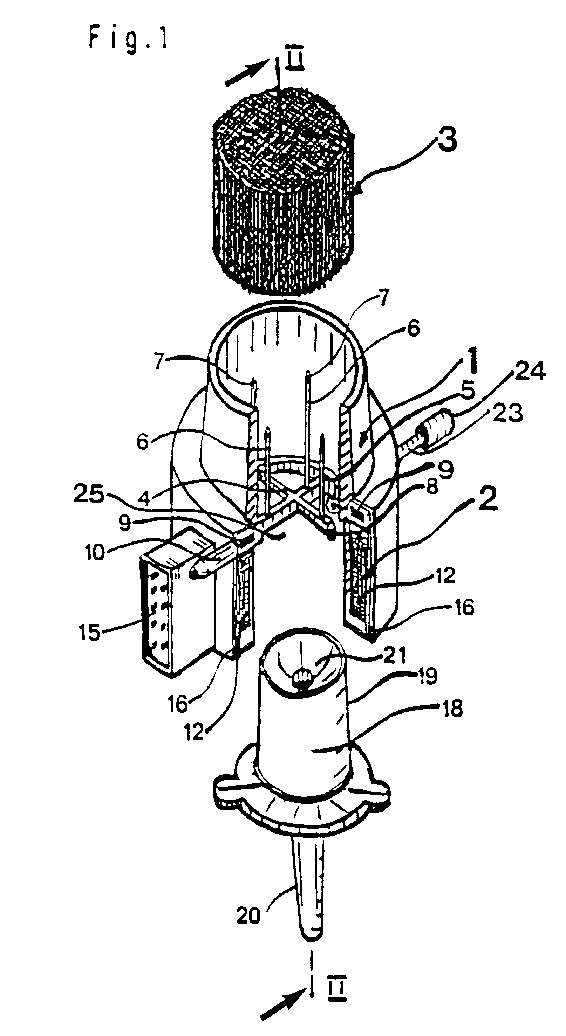

- Fig. 1:: eine perspektivische Explosionsdarstellung eines dreiteilig ausgeführten Ausführungsbeispiels der vorliegenden Erfindung und

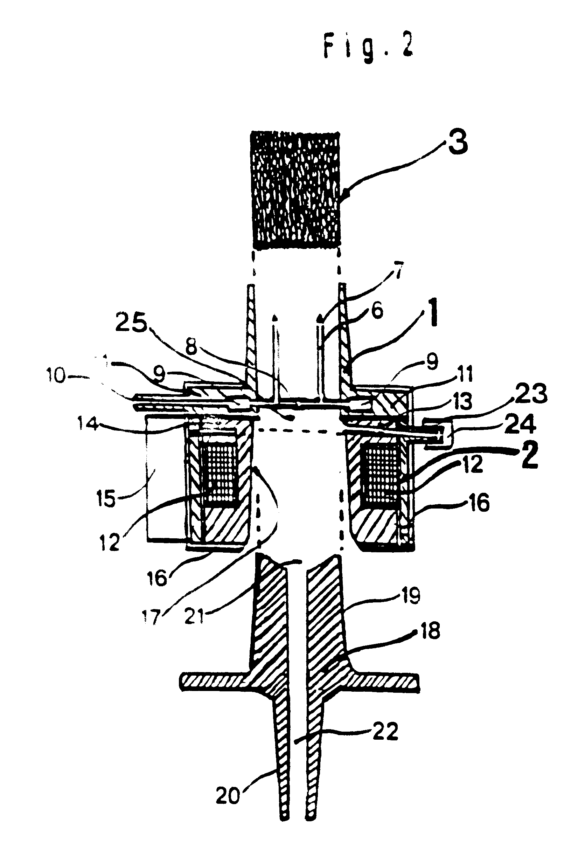

- Fig. 2:: einen Schnitt in Explosionsdarstellung entlang der Linie II-II durch die dreiteilige Vorrichtung gem. Fig. 1.

Die erfindungsgemäße Vorrichtung besteht aus 3 Teilen, wobei Teil 1 und Teil 2 überwiegend aus Metall bestehen und fest miteinander verbunden sind.

Teil 1 stellt ein Verbindungsstück zu einem Y-Adapter bzw. Beatmungskopf eines Beatmung-Schlauchsystems dar, über das die Atemgase in der Inspirationsphase zugeführt, in der Exspirationsphase abgeführt werden. Dieses Verbindungsstück 1 ist ausgebildet als Metall-Hohlzylinder, der außen mit einem Norm-Anschlußkonus versehen ist. In seinem Lumen wird vor Gebrauch ein satt passender, zylindrischer Wärme-Feuchte-Austauscher-Einsatz 3, der ein Tauschermedium, wie z.B. handelsübliche H.M.E. der Firma ICOR AB, Schweden, verwendet, eingeführt und durch den kreuzförmigen Einsatz 4 mit seinem Rand 5 abgestützt. Auf diesem Einsatz sind vier senkrecht stehende Metallröhrchen 6, die in Spitzen mit seitlichen Öffnungen 7 enden, angeordnet, Die Röhrchenenden reichen bis in das mittlere Drittel des Wärme-Feuchte-Austauscher-Einsatzes 3 und versorgen diesen exakt dosiert mit temperiertem Sterilwasser, das ihnen über eine externe bekannte Dosiervorrichtung über Schlauchstutzen 10, Ringkanal 9 und die Kanälchen 8 des kreuzförmigen Einsatzes 4 zugeführt wird. Der Ringkanal 9 ist über seine metallische Ummantelung 11 gut an den Heiz-Verbindungsteil 2 der Vorrichtung angekoppelt. Das in diesem Teil eingebaute Heizelement 12 und die zwei Temperatursensoren 13, 14 werden von einer externen Elektronik, die als solche schon bekannt ist, über ein Verbindungskabel und ein Vielfachstecker 15 mit Strom versorgt, sowie exakt kontrolliert und gesteuert. Der Kunststoffmantel 16 dient der Wärmeisolierung des Heizteils 2, der Norm-Innenkonus 17 der sicheren und zugleich gut lösbaren Verbindung des eingesteckten Tubusadapters 18 mit Norm-Außenkonus 19. Der Stutzen 23, der mit einer abnehmbaren Kappe 24 verschlossen ist verbindet den bei eingestecktem Tubusadapter 19 zwischen diesem und der Unterseite des kreuzförmigen Einsatzes 4 entstehenden flachen Hohlraum 25 mit dem Freien.

Nachfolgend wird die Funktion des hier anhand des Ausführungsbeispiels erläuterten Erfindungsgegenstandes beschrieben. Trockenes, kaltes Atemgas (ca. 20° Celsius, ca. 10% rel. Feuchte) gelangt während der Einatemphase, von einer in der Zeichnung nicht dargestellten Beatmungsmaschine über das Patientenschlauchsystem mit Y-Adapter zum Verbindungsstück 1. Von hier strömt das Atemgas durch den dort angeordneten Wärme-Feuchte-Austauscher-Einsatz 3 (H.M.E.-Einsatz). In seinem oberen Drittel, das überwiegend als passive H.M.E. wirkt, wird das kalte Atemgas in einer ersten Stufe passiv vorgewärmt und vorbefeuchtet durch gespeicherte Wärme und Feuchte, die zuvor der körperwarmen Ausatemluft des beatmeten Patienten entzogen worden war. Das Atemgas würde damit aber nur ca. 30° Celsius, 100% rel. Feuchte erreichen. Deswegen wird in einer zweiten Stufe in dem mittleren und unteren Drittel des H.M.E.-Einsatzes eine aktive Nachbefeuchtung und Nacherwärmung der Atemluft vorgenommen, so daß diese dann über die zentrale Bohrung 21, 22 des Tubusadapters 18 und über einen auf den Konus 20 aufgesteckten Trachealtubus in die Luftröhre des Patienten mit einer Temperatur von 36-37° Celsius bei 100% rel. Feuchte gelangt. Diese aktive Nachbefeuchtung und Nacherwärmung wird dabei in der Weise vorgenommen, daß über ein externes Steuergerät exakt dosiert Wasser und elektrische Heizenergie der obengenannten Vorrichtung zugeführt wird. Dies führt zu einer Erwärmung der metallenen Teile der Vorrichtung und vor allem aber durch das zusätzliche Austreten von erwärmtem Wasser über die Röhrchen 6 in das mittere und von dort sich vor allem der Schwere nach rasch ausbreitend das untere Drittel des H.M.E.-Einsatzes 3, zu deren aktiver Erwärmung und Befeuchtung. Durch die große H.M.E.-Einsatz-Oberfläche wird eine rasche, optimale Konditionierung der durchströmenden Einatemluft erreicht. In der Ausatemphase strömt das feuchte, körperwarme Atemgas (36-37° Celsius, 100% rel. Feuchte) über Tubus, Tubusadapter 18 zuerst in die aktiv befeuchteten und angewärmten unteren Zweidrittel des H.M.E.-Einsatzes 3, um dort nur wenig, anschließend in dessem oberem passivem Drittel 60-80% seiner gesamten Wärme und Feuchte abzugeben. Das bedeutet, daß in dem nachfolgenden Schlauchsystem nur noch eine geringe Feuchtigkeitsmenge als Kondensat ausfällt. Damit ergibt sich gegenüber konventionellen Systemen zusätzlich ein Vorteil, der sich nicht nur im pflegerischen Aufwand, sondern insbesondere auch in bakteriologisch-hygienischer Hinsicht zeigt.

Da bei der erfindungsgemäßen Vorrichtung der technisch komplexeste Teil, die verbundenen Teile 1 und 2 als leicht zu reinigende, dampfsterilisierbare, kombinierte, aktive Anwärm-Anfeucht-Vorrichtung mit Verbindungsstück für den Mehrmalgebrauch und der Wärme-Feuchte-Austauscher-Einsatz 3 in steriler, preiswerter Einmalgebrauchsausführung herstellbar ist, bedeutet dies beim Einsatz nicht nur eine optimale Konditionierung der Atemgase für den Patienten, sondern auch Schutz vor Infektionen bei hoher Wirtschaftlichkeit. Durch den Einbau von zwei Temperatursensoren mit nachgeschalteter zweikanaliger elektronischer Temperaturüberwachung und -regelung, einer Erwärmung der Anfeuchtvorrichtung nur wenig über Körpertemperatur und einem nur geringen Wärmespeichervermögen der Vorrichtung, läßt sich auch unter extremen Betriebsbedingungen eine Beatmung des Patienten mit zu heißen Atemgasen sicher vermeiden. Darüberhinaus ist durch das kleine Totraumvolumen des Anfeuchters ein gefährliches Rückatmen von Ausatemluft bei Säuglingen und Kindern nicht zu befürchten. In besonders schwierigen Beatmungssituationen z.B. wie bei der Frühgeborenenbeatmung mit hohen Frequenzen und kleinen Atemzugvolumina kann eine weitere funktionelle Totraumvolumen-Verringerung durch das Öffnen des Entlüftungstutzens 23 durch Abnehmen der Verschlußkappe 24 vorgenommen werden. Die verbrauchte Ausatemluft gelangt über den Hohlraum 25 und den dort mündenden Stutzen 23 dann direkt ins Freie.

## Patentansprüche

1. Vorrichtung zum Anwärmen und Anfeuchten von Gasen, insbesondere von Atemgasen bei der künstlichen Beatmung, bestehend aus einer Kombination von mindestens einem passiven Wärme-Feuchte-Austauscher (3) und mindestens einer aktiven Anwärmund Anfeuchtvorrichtung (12, 6)
**gekennzeichnet durch**
einen, in ein Gehäuse (1, 2) eingesetzten Wärme-Feuchte-Austauscher-Einsatz (3), der zu einem Teil als passiver Wärme-Feuchte-Austauscher arbeitet und zum anderen Teil zusammen mit einer ihn umgebenden Anwärm- und Anfeuchtvorrichtung (12, 6) als aktiver Wärme-Feuchte-Austauscher arbeitet.

2. Vorrichtung nach Anspruch 1, dadurch gekennzeichnet, daß das Gehäuse (1, 2) zweigeteilt ist, wobei der obere Gehäuseteil (1) als konisches Verbindungsstück mit einem zylindrischen Hohlraum zur Aufnahme des teilweise passiv wirkenden Wärme-Feuchte-Austauscher-Einsatzes (3) ausgebildet ist und wobei das untere Gehäuseteil (2) als aktive Anwärm- und Anfeuchtvorrichtung ausgebildet ist.

3. Vorrichtung nach Anspruch 1 oder 2, dadurch gekennzeichnet, daß zwischen dem Gehäuseteil (1) und dem Gehäuseteil (2) ein kreuzförmiger Einsatz (4) angeordnet ist, der von Kanälen (8) durchzogen ist, die einerseits mit einem Ringkanal (9) im Gehäuseteil (1) in Verbindung stehen und andererseits in auf dem kreuzförmigen Einsatz (4) senkrecht aufragenden Röhrchen (6) mit offenen Enden übergehen.

4. Vorrichtung nach Anspruch 3, dadurch gekennzeichnet, daß die Röhrchenenden (6) bis in das mittlere Drittel des WärmeFeuchte-Austauscher-Einsatzes (3) ragen.

5. Vorrichtung nach Anspruch 3 oder 4, dadurch gekennzeichnet, daß die Arme des kreuzförmigen Einsatzes ein strömungsgünstiges Profil durch Verschmälerungen und Abrundungen an ihren Ober- und Unterseiten aufweisen.

6. Vorrichtung nach einem der Ansprüche 1-5, dadurch gekennzeichnet, daß die Gehäuseteile (1, 2) aus Metall bestehen und zumindest teilweise mit einem wärmeisolierenden Kunststoffmantel (16) überzogen sind.

7. Vorrichtung nach einem der Ansprüche 3-6, dadurch gekennzeichnet, daß das Gehäuseteil (2) einen Tubusadapter (18) einsetzbar aufnimmt.

8. Vorrichtung nach einem der Ansprüche 1-7, dadurch gekennzeichnet, daß das Gehäuseteil (2) einen Entlüftungsstutzen (23) ins Freie aufweist, der mit einer abnehmbaren Kappe (24) verschließbar ist.

## Claims

1. Apparatus for pre-heating and moistening gases, especially respiratory gases in artificial respiration, comprising a combination of at least one passive heat-moisture exchanger (3) and at least one active pre-heating and moistening apparatus (12, 6),
characterized by
a heat-moisture exchanger insert (3) which is inserted into a housing (1, 2) and operates, for the one part, as a passive heat-moisture exchanger and, for the other part, operates together with a pre-heating and moistening apparatus (12, 6) surrounding it as an active heat-moisture exchanger.

2. Apparatus according to Claim 1, characterized in that the housing (1, 2) is divided in two, the upper housing part (1) being configured as a conical connecting piece having a cylindrical cavity for receiving the partly passively functioning heat-moisture exchanger insert (3) and the lower housing part (2) being configured as an active pre-heating and moistening apparatus.

3. Apparatus according to Claim 1 or 2, characterized in that between the housing part (1) and the housing part (2) there is disposed a cruciform insert (4) which is passed through by ducts (8) which, on the one hand, are connected to an annular duct (9) in the housing part (1) and, on the other hand, merge into open-ended tubes (6) jutting vertically up on the cruciform insert (4).

4. Apparatus according to Claim 3, characterized in that the tube ends (6) jut right into the middle third of the heat-moisture exchanger insert (3).

5. Apparatus according to Claim 3 or 4, characterized in that the arms of the cruciform insert exhibit a flow-favourable profile by virtue of constrictions and roundings on their top and bottom sides.

6. Apparatus according to one of Claims 1-5, characterized in that the housing parts (1, 2) consist of metal and are coated, at least partially, with a heat-insulating plastics casing (16).

7. Apparatus according to one of Claims 3-6, characterized in that the housing part (2) receives, in an insertable manner, a tube adapter (18).

8. Apparatus according to one of Claims 1-7, characterized in that the housing part (2) exhibits an air-vent connection (23) into the open, which can be closed by means of a removable cap (24).

## Revendications

1. Dispositif pour chauffer et humidifier des gaz, notamment de gaz de respiration pour une respiration artificielle, constitué d'une combinaison comprenant au moins un échangeur passif de chaleur-humidité (3) et au moins un dispositif actif d'échauffement et d'humidification (12,6), caractérisé par un insert d'échangeur de chaleur-humidité (3) placé dans un boîtier (1,2) qui fonctionne en partie comme échangeur passif de chaleur-humidité et qui fonctionne en partie conjointement avec un dispositif d'échauffement et d'humidification (12,6) entourant celui-ci comme échangeur actif de chaleur-humidité.

2. Dispositif selon la revendication 1, caractérisé en ce que le boîtier (1,2) est divisé en deux parties, la partie supérieure de boîtier (1) étant réalisée sous forme de pièce de liaison conique avec un espace creux cylindrique pour la réception de l'insert d'échangeur de chaleur-humidité (3) agissant partiellement de manière passive, et dans lequel la partie inférieure de boîtier (2) est réalisée sous forme de dispositif actif d'échauffement et d'humidification.

3. Dispositif selon la revendication 1 ou 2, caractérisé en ce qu'il est disposé entre la partie de boîtier (1) et la partie de boîtier (2) un insert en forme de croix (4) qui est traversé par des canaux (8) qui communiquent d'une part avec un canal annulaire (9) dans la partie de boîtier (1) et qui rejoignent d'autre part des petits tubes (6) à extrémités ouvertes faisant saillie verticalement sur l'insert cruciforme (4).

4. Dispositif selon la revendication 3, caractérisé en ce que les extrémités (6) des petits tubes font saillie jusque dans le tiers moyen de l'insert d'échangeur de chaleurhumidité (3).

5. Dispositif selon la revendication 3 ou 4, caractérisé en ce que les bras de l'insert cruciforme ont un profil favorisant le courant par des rétrécissements et des arrondis à leurs côtés supérieurs et inférieurs.

6. Dispositif selon l'une des revendications 1 à 5, caractérisé en ce que les parties de boîtier (1,2) sont en métal et sont recouvertes au moins partiellement d'une enveloppe (16) en matière synthétique calorifuge.

7. Dispositif selon l'une des revendications 3 à 6, caractérisé en ce qu'un adaptateur de tube (18) peut être placé sur la partie de boîtier (2).

8. Dispositif selon l'une des revendications 1 à 7, caractérisé en ce que la partie de boîtier (2) présente une tubulure d'aération (23) vers l'extérieur qui peut être fermée par un capuchon amovible (24).
